# EUROPEAN PATENT APPLICATION

(11) **EP 4 656 072 A1**
(43) Date of publication of application: **03.12.2025**
(21) Application number: 25176265.4
(22) Date of filing: 14.05.2025
(51) Int. Cl.: A24F 40/30, A24F 40/10, A24F 40/485, A24F 40/50, A61M 15/06

(54) **ATOMIZATION ASSEMBLY AND ATOMIZATION DEVICE**

(30) Priority: 14.05.2024 CN 202421049709 U
(71) Applicant: Shenzhen Relx Technology Co., Ltd., Shenzhen City, Guangdong Province 518108 (CN)
(72) Inventor: XU, SHENGYANG, Shenzhen, 518108 (CN); CHEN, ZHEN, Shenzhen, 518108 (CN); GUO, HAOHANG, Shenzhen, 518108 (CN)
(74) Representative: Metida

(57) **Abstract**

An atomization assembly (100) and device are provided, including a main shell (10), a mouthpiece (20), at least two electrode assemblies (30), a power supply component (40), and a circuit switching component (50). The main shell (10) is provided with at least two receiving chambers (11) each for receiving a liquid storage component (200). The mouthpiece (20) is connected to an end (10a) of the main shell (10) and provided with an inhalation port (P0). The inhalation port (P0) corresponds to the liquid storage component (200) in at least one receiving chamber (11). The electrode assemblies (30) correspond and are electrically connected to the liquid storage components (200). The circuit switching component (50) selectively connects the power supply component (40) to one electrode assembly (30), thereby establishing an electrical connection between the power supply component (40) and the liquid storage component (200) corresponding to the inhalation port (P0). The present application can meet the requirements for different flavors and large capacity of smoke and improve the convenience of user operation.

## Description

### FIELD

The present application relates to the field of atomization and delivery of an aerosolizable substance into a human body, and more particularly, to an atomization assembly and an atomization device having the atomization assembly.

### BACKGROUND

Atomization devices are electronic products that imitate cigarettes, which can turn nicotine or e-liquid into smoke that the user can inhale by atomization and other means. In related arts, the atomization device may include a cartridge. Such cartridge uses only one type of e-liquid at a time, and the amount of smoke is small, making it difficult to meet the requirements for different flavors and large capacity of smoke. Therefore, the users need to carry other cartridges with them for replacement, which is inconvenient.

### SUMMARY

In view of the above shortcomings, an atomization assembly and atomization device are needed.

A first aspect of the present application provides an atomization assembly, including a main shell, a mouthpiece, at least two electrode assemblies, a power supply component, and a circuit switching component. The main shell includes a first end and a second end arranged opposite to the first end along a length direction of the atomization assembly. The main shell is provided with at least two receiving chambers, and each of the at least two receiving chambers is configured to receive a liquid storage component therein. The mouthpiece is connected to the first end and provided with a inhalation port. The inhalation port is configured to correspond to the liquid storage component received at least one of the receiving chambers. The at least two electrode assemblies are configured to correspond one-to-one to and electrically connected to the liquid storage components in the at least two receiving chambers. The circuit switching component is electrically connected to the power supply component. The circuit switching component selectively connects the power supply component to one of the at least two electrode assemblies, thereby establishing an electrical connection between the power supply component and the liquid storage component corresponding to the inhalation port.

Based on the first aspect, in some possible implementations, the mouthpiece is movably connected to the first end. The liquid storage component is provided with a first air passage. The inhalation port selectively communicates with the first air passage of the liquid storage component in one of the at least two receiving chambers when the mouthpiece moves relative to the main shell.

Based on the first aspect, in some possible implementations, the mouthpiece is fixed to the first end. The liquid storage component is provided with a first air passage. When viewed along the length direction, the inhalation port is configured to cover the first air passages of the liquid storage components in the at least two receiving chambers.

Based on the first aspect, in some possible implementations, the circuit switching component includes at least two airflow switches and a circuit board. The at least two airflow switches are arranged to correspond one-to-one to the at least two receiving chambers. The at least two airflow switches are configured to detect a change of airflow or pressure in the first air passages of the liquid storage components in the at least two receiving chambers, respectively. The electrode assemblies are electrically connected to the circuit board. The circuit board is further electrically connected to the power supply components and the at least two airflow switches. The circuit board is configured to electrically connect the power supply component to one of the at least two electrode assemblies according to a detection result of the airflow switches.

Based on the first aspect, in some possible implementations, the circuit switching component includes an airflow switch, a circuit board, and a mechanical switch. The airflow switch is configured to detect a change of airflow or pressure in the first air passages of the liquid storage components in the at least two receiving chambers. The electrode assembly is electrically connected to the circuit board. The circuit board is further electrically connected to the power supply components. The mechanical switch is connected to the circuit board and configured to switch between at least two positions. The circuit board is configured to electrically connect the power supply component to one of the at least two electrode assemblies based on a detection result of the airflow switch and the position of the mechanical switch.

Based on the first aspect, in some possible implementations, the circuit switching component includes an airflow switch, an electrical connection component, a circuit board, and at least two electrodes. The airflow switch is configured to detect a change of airflow or pressure in the first air passages of the liquid storage components in the at least two receiving chambers. The electrical connection component is located in the mouthpiece. The electrode assembly is electrically connected to the circuit board. The circuit board is further electrically connected to the power supply component. The at least two electrodes are installed on the circuit board and extend towards the electrical connection component. The electrical connection component selectively conducts at least one of the electrodes when the mouthpiece moves relative to the main shell. The circuit board is configured to electrically connect the power supply component to one of the at least two electrode assemblies according to the detection result of the airflow switch and the electrode which is connected to the electrical connection component.

Based on the first aspect, in some possible implementations, the power supply component is disposed within the main shell and located between the at least two receiving chambers. The atomization assembly further includes a bottom shell detachably connected to the second end. The electrode assemblies and the circuit switching component are located at a side of the bottom shell facing the mouthpiece.

Based on the first aspect, in some possible implementations, each of the power supply component, the electrode assemblies, and the circuit switching component is disposed within the main shell and located between the at least two receiving chambers. The main shell is further provided with at least two first air branch passages. Each of the at least two first air branch passages includes a first end portion and a second end portion. The first end portion is configured to communicate with the first air passage of the liquid storage component in one of the at least two receiving chambers. The second end is connected to one of the at least two airflow switches.

Based on the first aspect, in some possible implementations, the atomization assembly further includes a bottom shell detachably connected to the second end. Each of the power supply component, the electrode assemblies, and the circuit switching component is located in the bottom shell. The main shell is further provided with a first air branch passage. The first air branch passage is configured to communicate with the first air passages of the liquid storage components in the at least two receiving chambers. The bottom shell is provided with a second air branch passage communicating with the first air branch passage. The second air branch passage is further connected to the airflow switch.

Based on the first aspect, in some possible implementations, the mouthpiece is provided with a first magnetic component. The main shell is provided with a second magnetic component. The first magnetic component is detachably connected to the second magnetic component.

Based on the first aspect, in some possible implementations, the mouthpiece is provided with one of a buckle and a slot. The main shell is provided with the other of the buckle and the slot. The buckle is detachably connected to the slot.

Based on the first aspect, in some possible implementations, the power supply component is located in the main shell. Along a width direction perpendicular to the length direction, the power supply component is located at a side of the at least two receiving chambers. The atomization assembly further includes a bottom shell detachably connected to the second end. The electrode assemblies and the circuit switching component are located at a side of the bottom shell facing the mouthpiece.

The second aspect of the present application provides an atomization device, which includes the atomization assembly as described above and at least two liquid storage components. The at least two liquid storage components are located in the at least two receiving chambers of the atomization assembly, respectively.

The atomization device of the present application can simultaneously receive at least two liquid storage components therein, and at least two electrode assemblies correspond one-to-one to and electrically connected to the at least two liquid storage components. The circuit switching component can selectively connect the power supply component to one of the electrode assemblies, such that the power supply component can be electrically connected to the liquid storage component connected to the inhalation port through the electrode assembly (i.e., switch between different liquid storage components to operate), thereby heating and atomizing the aerosolizable substance in the liquid storage component into an aerosol that can be inhaled by the user. Therefore, even when the user does not carry other liquid storage components with them for replacement, the present application can meet requirements for different flavors and large capacity of smoke and improve the convenience of user operation. In addition, due to the setting of the at least two electrode assemblies, when it is needed to switch between different liquid storage components, it has no need to simultaneously switch between the electrode assemblies such as by rotation, sliding, or other means, which can reduce the damages to the electrode assemblies and further improving the convenience of user operation.

### BRIEF DESCRIPTION OF THE DRAWINGS

FIG. 1 is a diagrammatic view of an atomization device according to an embodiment of the present application;
FIG. 2 is a diagrammatic view showing the atomization device in FIG. 1 from another angle;
FIG. 3 is a diagrammatic view showing the atomization device in FIG. 1 when a protective cover is opened;
FIG. 4 is a diagrammatic view showing the atomization device in FIG. 1 when the protective cover and a main shell are removed;
FIG. 5 is a sectional view of the atomization device taken along V-V in FIG. 1;
FIG. 6 is a sectional view showing the atomization device in FIG. 1 when switched to another state;
FIG. 7 is a sectional view of an atomization assembly when a liquid storage component of the atomization device in FIG. 5 is removed;
FIG. 8 is a simplified diagram of the atomization device when viewed from a length direction ;
FIG. 9 is a diagrammatic view of atomization devices according to another embodiment of the present application;
FIG. 10 is a cross-sectional view of the atomization device shown in FIG. 9 taken along X-X in FIG. 9;
FIG. 11 is a diagrammatic view of an atomization devices according to another embodiment of the present application;
FIG. 12 is a diagrammatic view showing the atomization device in FIG. 11 when switched to another state;
FIG. 13 is a cross-sectional view of an atomization device according to another embodiment of the present application;
FIG. 14 is a cross-sectional view of an atomization device according to another embodiment of the present application;
FIG. 15 is a cross-sectional view of an atomization device according to another embodiment of the present application.

### DETAILED DESCRIPTION

Implementations of the present technology will now be described, by way of embodiment, with reference to the attached figures. The embodiments are obviously a portion but not all of the embodiments of the present application. Unless otherwise defined, the technical and scientific terms used in the description have the same meanings as those commonly understood by one skilled in the art. The terms used in the description are for the purpose of describing specific embodiments but not intended to limit the scope of the present application.

The following description will provide a detailed explanation of the embodiments of the present application. However, the present application may also be presented in other forms and not limited by the exemplary embodiments illustrated herein.

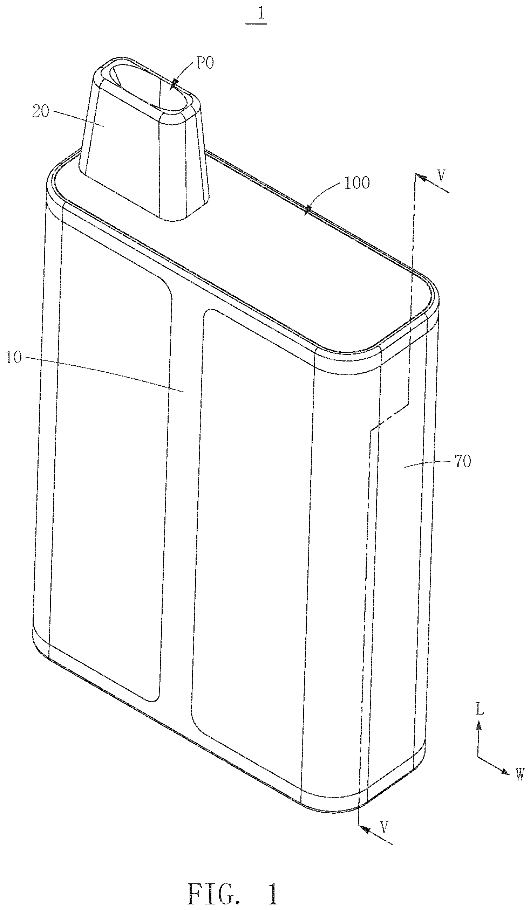

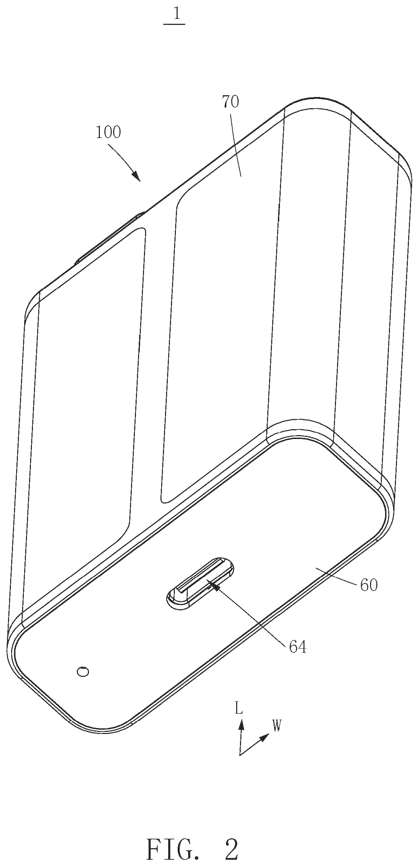

Referring to FIGS. 1 to 3, an atomization device 1 is provided according to an embodiment of the present application. The atomization device 1 can atomize an aerosolizable substance into an aerosol for users to inhale. The atomization device 1 may be an electronic cigarette, and correspondingly, the aerosolizable substance may be an e-liquid.

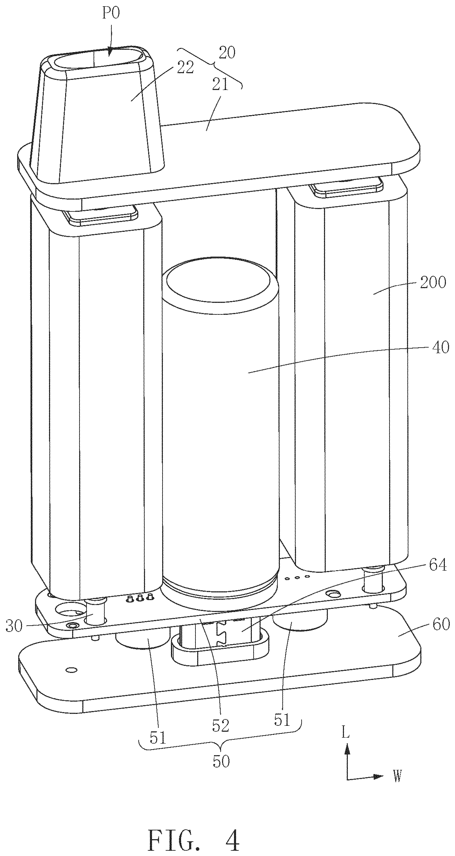

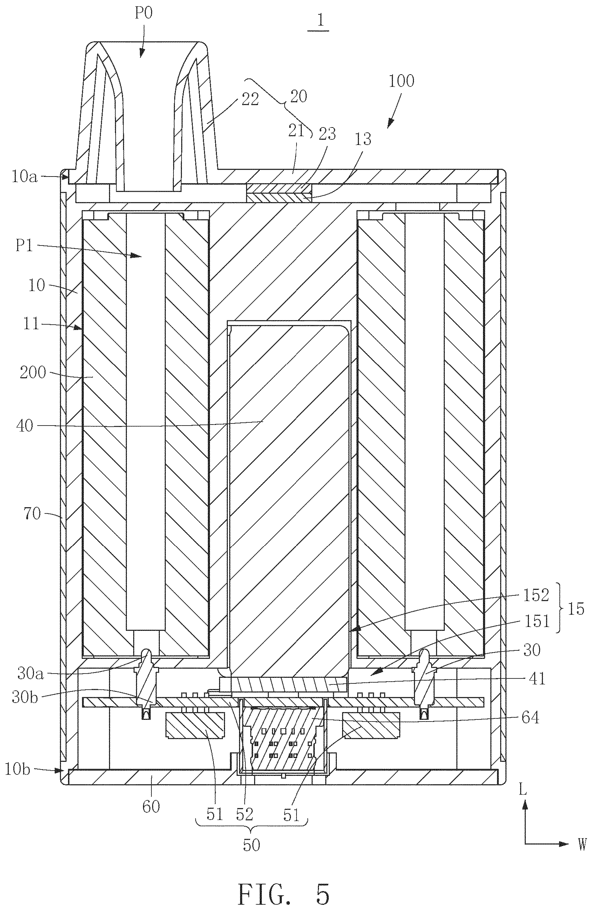

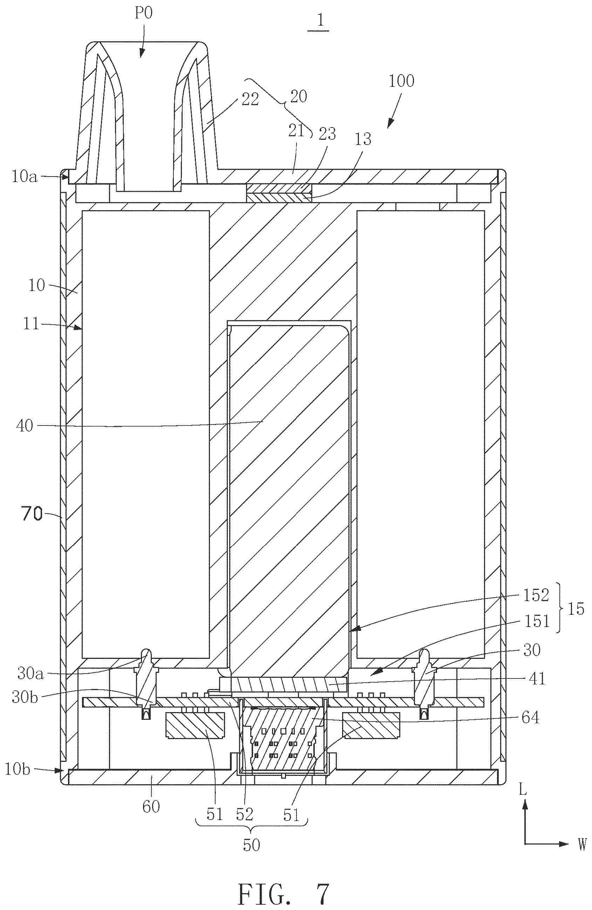

Also referring to FIGS. 4 to 7, the atomization device 1 includes an atomization assembly 100 and at least two liquid storage components 200. FIG. 7 is a cross-sectional view of the atomization assembly 100 when the liquid storage components 200 are removed from the atomization device 1 shown in FIG. 5. The atomization assembly 100 includes a main shell 10, a mouthpiece 20, at least two electrode assemblies 30, and a power supply component 40.

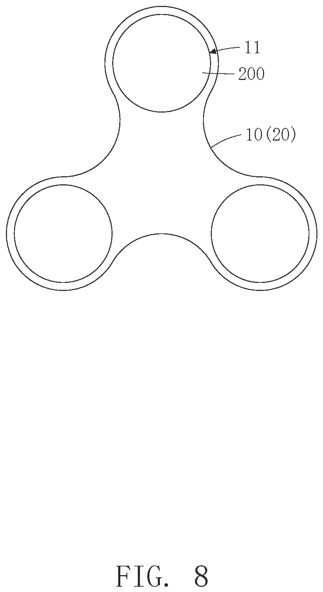

The atomization assembly 100 has a length direction L, and the main shell 10 includes a first end 10a and a second end 10b arranged opposite to the first end 10a along the length direction L. The main shell 10 is provided with at least two receiving chambers 11. Each receiving chamber 11 can receive one liquid storage component 200 such as a cartridge therein. In some embodiments, the main shell 10 is provided with two receiving chambers 11, indicating that the main shell 10 can receive two liquid storage components 200 therein. At this time, a cross-section of the main shell 10 (hereinafter, the cross-section refers to a plane perpendicular to the length direction L) is substantially rectangular. As shown in FIG. 8, in other embodiments, the main shell 10 may also define more than two receiving chambers 11. For example, the main shell 10 is provided with three receiving chambers 11, indicating that the main shell 10 can receive three liquid storage components 200 therein. At this time, the cross-section of the main shell 10 may substantially triangular with arc sides. A cross-section of the receiving chamber 11 may be circular or square. The cross-sections of the receiving chambers 11 may be the same or different to each other, that is, the main shell 10 can receive at least two liquid storage components 200 of different shapes. The type of the aerosolizable substance in each liquid storage component 200 may be the same, such that multiple liquid storage components 200 can increase the capacity of the atomization device 1. The type of the aerosolizable substance in each liquid storage component 200 may also be different from each other, such that the atomization device 1 can meet the requirements of various flavors. The atomization assembly 100 further has a width direction W perpendicular to the length direction L. When there are two receiving chambers 11, the width direction W may be the arrangement direction of the two receiving chambers 11. When there are three receiving chambers 11, the width direction W may be any direction perpendicular to the length direction L.

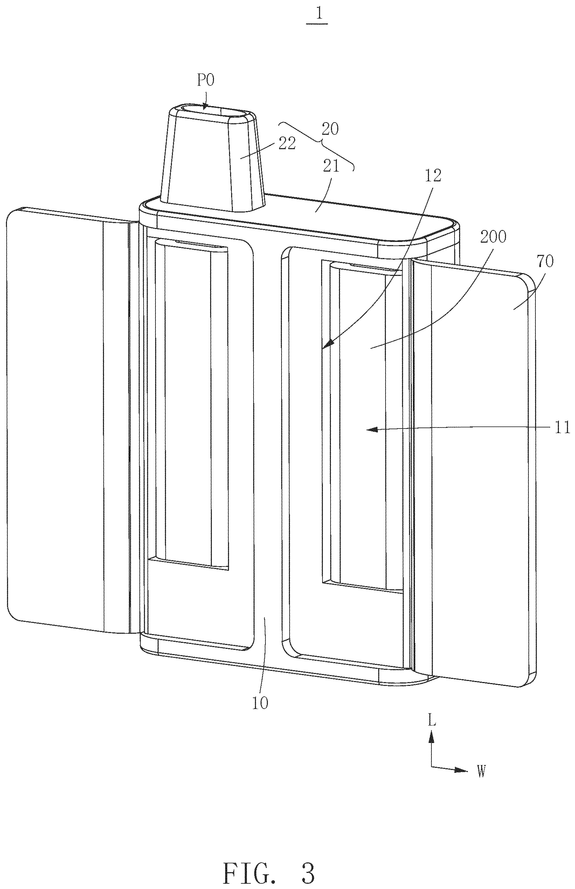

Furthermore, as shown in FIG. 3, the sidewall of the main shell 10 may further define at least two first openings 12. The at least two first openings 12 correspond one-to-one to the at least two receiving chambers 11. Each receiving chamber 11 is exposed from one first opening 12, such that the user can replace the liquid storage component 200 in the receiving chamber 11. For example, the user can remove the old liquid storage component 200 from the receiving chamber 11 through the first opening 12 and install a new liquid storage component 200 into the receiving chamber 11 through the first opening 12. In at least one embodiment, a buckle may be provided in the receiving chamber 11, and correspondingly, a slot may be provided on the liquid storage component 200. The engagement between the buckle and the slot allows the liquid storage component 200 to be detachably installed in the receiving chamber 11, thereby reducing the risk of the liquid storage component 200 separating from the receiving chamber **11** during use. In other embodiments, the liquid storage component 200 may also be detachably installed in the receiving chamber 11 through interference fit or other manners, which is not limited in the present application. As shown in FIGS. 1 to 3, the atomization assembly 100 may further include a protective cover 70 sleeved on the main shell 10. When the atomization device 1 is in use, the protective cover 70 covers the first openings 12. The first opening 12 may also be exposed from the protective cover 70, thereby allowing the user to replace the liquid storage components 200. For example, the protective cover 70 is first removed, and then, the user can remove the old liquid storage component 200 from the receiving chamber 11 through the first opening 12 and install the new liquid storage component 200 into the receiving chamber 11 through the first opening 12.

As shown in FIG. 5, the liquid storage component 200 is provided with a first air passage P1. In some embodiments, the liquid storage component 200 includes a liquid storage portion and a heating portion. The liquid storage portion can store an aerosolizable substance such as the e-liquid. The liquid storage portion is substantially circular and is provided with the first air passage P1. The heating portion is located in and in contact with the liquid storage portion. The heating portion can heat and atomize the aerosolizable substance in the liquid storage portion into aerosol that can be inhaled by the user. In some embodiments, the liquid storage portion may be a liquid storage cotton. The heating portion may be a heating mesh or a heating wire, and includes a first pin and a second pin.

The mouthpiece 20 is connected to the first end 10a. The mouthpiece 20 is provided with an inhalation port P0 therein. The inhalation port P0 can correspond to the liquid storage component 200 in at least one of the receiving chambers 11. For example, the inhalation port P0 can communicate with the first air passage P1 of the liquid storage component 200 in at least one receiving chamber 11, thereby forming an airflow channel. Therefore, the aerosol formed by heating and atomizing the aerosolizable substance can be delivered through the airflow channel for users to inhale. In some embodiments, the mouthpiece 20 includes an installation portion 21 and a mouthpiece portion 22 integrally formed with the installation portion 21. The installation portion 21 is movably connected to the first end 10a of the main shell 10. The mouthpiece portion 22 protrudes from a surface of the installation portion 21 away from the main shell 10. The inhalation port P0 extends through the mouthpiece portion 22 and the installation portion 21. The user can inhale at the mouthpiece portion 22, such that the aerosol is delivered through the first air passage P1 and inhaled by the user at the inhalation port P0. The shape of the mouthpiece portion 22 may be duckbill, circular, and elliptical that satisfies ergonomics.

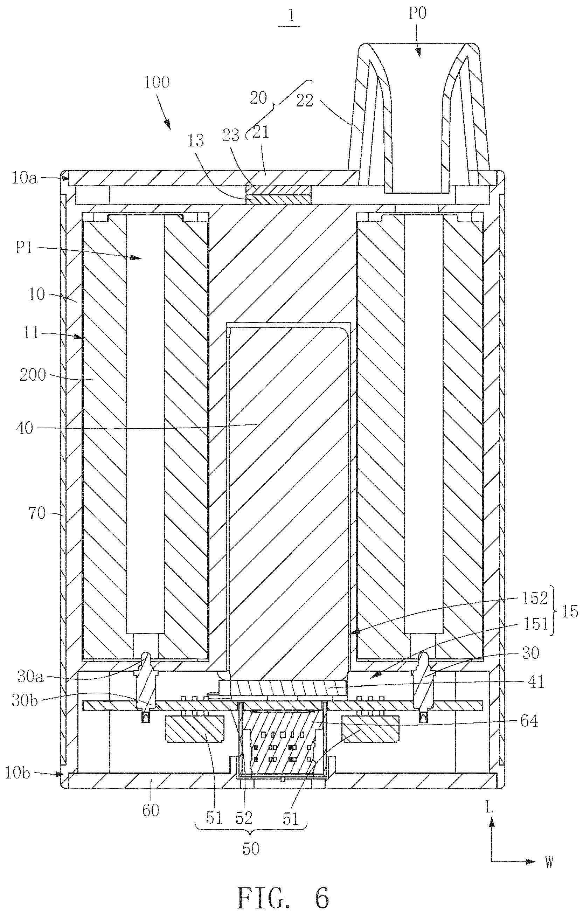

In some embodiments, the mouthpiece 20 is movably connected to the first end 10a. For example, the mouthpiece 20 is detachably or rotatably connected to the first end 10a. Taking detachable connection between the mouthpiece 20 and the first end 10a for example, as shown in FIGS. 5 and 6, the mouthpiece 20 can be removed from the first end 10a, rotated by a certain angle, and then connected to the first end 10a again. When the mouthpiece 20 is rotatably connected to the first end 10a, the mouthpiece 20 may be provided with a rotating shaft, and the main shell 10 may be provided with a main shell seat rotatably connected to the rotating shaft. When the mouthpiece 20 moves relative to the main shell 10, the inhalation port P0 can selectively communicate with the first air passage P1 of one liquid storage component 200. In some embodiments, there are two receiving chambers 11, and the inhalation port P0 first communicates with the first air passage P1 of one liquid storage component 200; when the mouthpiece 20 is removed from the first end 10a, rotated substantially by 180 degrees, and connected to the first end 10a again, the inhalation port P0 can communicate with the first air passage P1 of another liquid storage component 200, thereby switching between different liquid storage components 200 for operation. As shown in FIG. 8, in other embodiments, there are three receiving chambers 11, and the inhalation port P0 first communicates with the first air passage P1 of one liquid storage components 200; when the mouthpiece 20 is removed from the first end 10a, rotated substantially by 120 degrees, and connected to the first end 10a again, the inhalation port P0 can communicate with the first air passage P1 of another liquid storage component 200.

As shown in FIGS. 5 and 6, in some embodiments, the mouthpiece 20 is provided with a first magnetic component 23, and the main shell 10 is provided with a second magnetic component 13 at the first end 10a. The first magnetic component 23 is detachably connected to the second magnetic component 13, such that the mouthpiece 20 is detachably connected to the first end 10a. In another embodiment, the mouthpiece 20 may also be provided with a buckle (not shown), and the main shell 10 may also be provided with a slot (not shown) at the first end 10a. The buckle is detachably engaged with the slot, such that the mouthpiece 20 is detachably connected to the first end 10a. It can be understood that in other embodiments, the mouthpiece 20 may also be provided with a slot, and the main shell 10 may also be provided with a buckle at the first end 10a. At this time, the engagement of the buckle and the slot also allows the mouthpiece 20 to be detachably connected to the first end 10a.

The electrode assemblies 30 correspond one-to-one to the liquid storage components 200 in the receiving chambers 11. Each electrode assembly 30 can be electrically connected to one corresponding liquid storage component 200. In some embodiments, each electrode assembly 30 includes a pair of electrodes, and each electrode includes a first conductive end 30a and a second conductive end 30b arranged opposite to the first conductive end 30a. Each electrode assembly 30 shows only one electrode in FIGS. 5 and 6, since the two electrodes of each electrode assembly 30 overlaps with each other.

As shown in FIGS. 5 and 6, the atomization assembly 100 further includes a circuit switching component 50. The circuit switching component 50 is electrically connected to each electrode assembly 30 and the power supply component 40. The circuit switching component 50 can selectively connect the power supply component 40 to one of the electrode assemblies 30, thereby establishing an electrical connection between the power supply component 40 and the liquid storage component 200 communicating with the inhalation port P0. Therefore, the power supply component 40 can supply electric power to the liquid storage component 200, and the heating portion heats and atomizes the aerosolizable substance of the liquid storage component 200, thereby forming the aerosol that can be inhaled by the user. Therefore, even when the user does not carry other liquid storage components with them for replacement, the present application can meet requirements for different flavors and large capacity of smoke and improve the convenience of user operation. In addition, due to the setting of the at least two electrode assemblies 30, when it is needed to switch between different liquid storage components 200, it has no need to simultaneously switch between different electrode assemblies 30 such as by rotation, sliding, or other means, which can reduce the damages to the electrode assemblies 30 and further improving the convenience of user operation. The power supply component 40 includes a first electrode and a second electrode with opposite polarities. In each electrode assembly 30, the first conductive end 30a of one electrode can be electrically connected to the first pin of the heating portion, and the second conductive end 30a of the electrode can be electrically connected to the first electrode of the power supply component 40 through the circuit switching component 50; the first conductive end 30a of another electrode can be electrically connected to the second pin of the heating portion, and the second conductive end 30a of the electrode can be electrically connected to the second electrode of the power supply component 40 through the circuit switching component 50. Thus, the power supply component 40 can supply electric power to the liquid storage component 200 communicating with the inhalation port P0 through the electrode assembly 30. Each electrode assembly 30 may be a pair of conductive PIN needles made of metal materials. In some embodiments, a connector 64 may be provided on the bottom shell 60 and electrically connected to the power supply component 40. The connector 64 can be connected to an external power source to charge the power supply component 40. The connector 64 may be a Universal Serial Bus (USB) charging connector.

As shown in FIGS. 5 and 6, in some embodiments, the power supply component 40 is disposed within the main shell 10 and located between the receiving chambers 11. The atomization assembly 100 further includes a bottom shell 60 detachably connected to the second end 10b. The electrode assemblies 30 and the circuit switching component 50 are located at a side of the bottom shell 60 facing the mouthpiece 20. For example, the main shell 10 is further provided with a receiving space 15. The receiving space 15 is divided into a first receiving area 151 and a second receiving area 152 communicating with the first receiving area 151. The first receiving area 151 is located at a side of the receiving chambers 11 along the length direction L, and also at the side of the bottom shell 60 facing the mouthpiece 20. The first receiving area 151 is closer to the second end 10b than the receiving chambers 11. The second receiving area 152 is located between the receiving chambers 11. The power supply component 40 is located in the second receiving area 152. The electrode assemblies 30 and the circuit switching component 50 are located in the first receiving area 151, and the first conductive ends 30a of each electrode assembly 30 extend from the first receiving area 151 into the corresponding receiving chamber 11 to electrically connect to the liquid storage component 200. As such, the internal structure of the atomization device 1 may be compact, thereby improving the space utilization. The bottom shell 60 may be fixed to or detachably connected to the second end 10b. The bottom shell 60 covers the first receiving area 151 to protect the electrode assemblies 30 and the circuit switching component 50. The bottom shell 60 may substantially a flat plate.

As shown in FIGS. 5 and 6, the circuit switching component 50 may include at least two airflow switches 51 and a circuit board 52. The airflow switches 51 correspond one-to-one to the receiving chambers 11, and each airflow switch 51 may substantially be located at a side of the first air passage P1 of the corresponding liquid storage component 200 away from the inhalation port P0. Each airflow switch 51 can detect a change of airflow or pressure in the airflow channel formed by the inhalation port P0 and the first air passage P0 of the corresponding liquid storage component 200. When the user inhales at the inhalation port P0 of the mouthpiece 20, the first air passage P1 of one liquid storage component 200 communicates with the inhalation port P0 to form the airflow channel. Therefore, one airflow switch 51 can detect a change of airflow or pressure in the above airflow channel. The electrode assemblies 30 are electrically connected to the circuit board 52 and may be installed on the circuit board 52. The second conductive ends 30b of the electrode assemblies 30 may be electrically connected to the circuit board 52. The circuit board 52 is further electrically connected to the power supply component 40 and the airflow switches 51. When one airflow switch 51 detects a change of airflow or pressure, the electrical signal generated by the airflow switch 51 causes the circuit board 52 to electrically connect the power supply component 40 to one electrode assembly 30, thereby electrically connecting the power supply component 40 to one liquid storage component 200 communicating with the inhalation port P0 through the electrode assembly 30. In some embodiments, the pair of electrodes of the electrode assembly 30 are telescopic relative to the circuit board 52 (e.g., the electrodes may be spring pins). When the liquid storage components 200 are placed in the receiving chambers 11, the pair of electrodes of each electrode assembly 30 extend into the corresponding receiving chamber 11 and resist against the corresponding liquid storage component 200. The airflow switch 51 may be a microphone, and the power supply component 40 may be a cylindrical battery. In some embodiments, the atomization assembly 100 further includes a buffer component 41 located in the second receiving area 152. The buffer component 41 is located between power supply component 40 and the circuit board 52 along the length direction L, and can provide buffer functions against collisions between the power supply component 40 and other components.

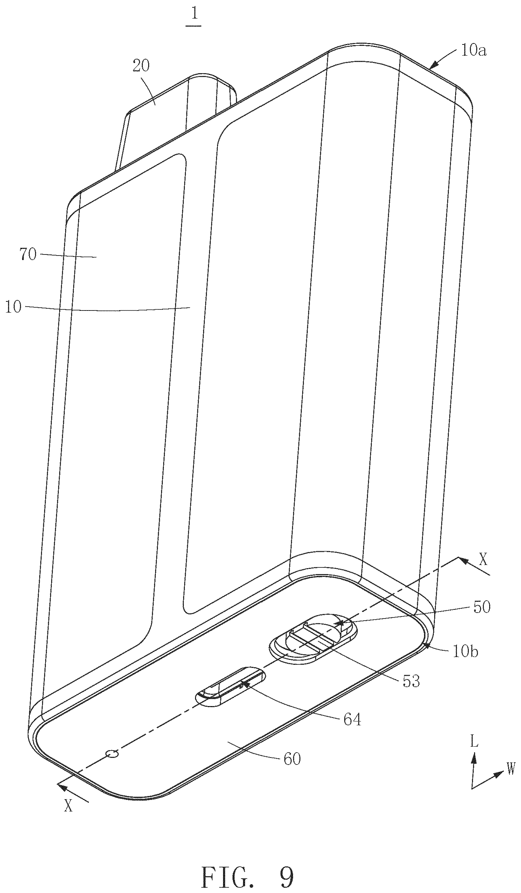

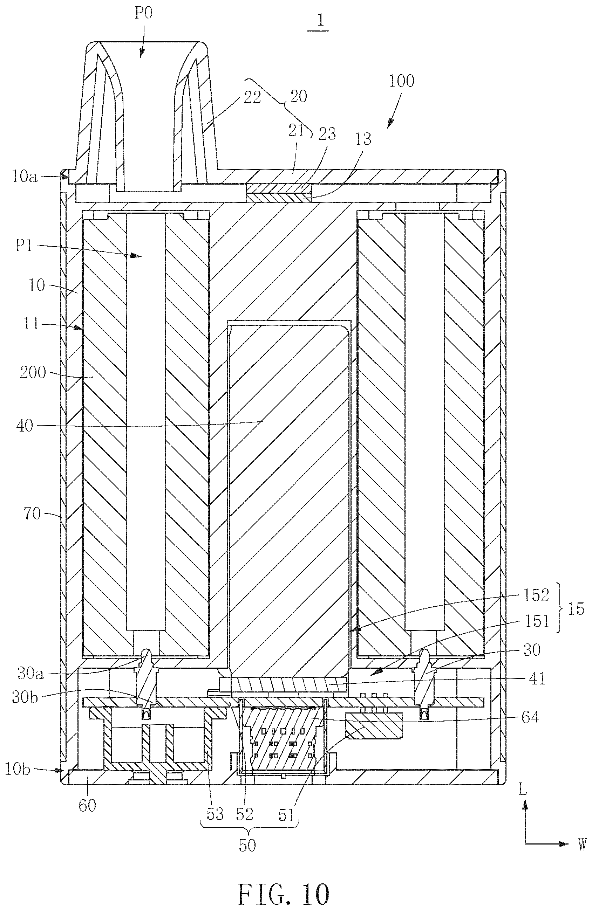

The structure of the circuit switching component 50 may also be changed. As shown in FIGS. 9 and 10, in another embodiment, the circuit switching component 50 may further include an airflow switch 51, a circuit board 52, and a mechanical switch 53. There is only one airflow switch 51, which simultaneously communicates with the first air passage P1 of each liquid storage component 200. For example, the airflow switch 51 may be located in the first receiving area 151, and the first receiving area 151 communicates with each receiving chamber 11. When the user inhales at the inhalation port P0 of the mouthpiece 20, the airflow or pressure in the first receiving area 151 changes, such that the airflow switch 51 can detect a change of airflow or pressure. The electrode assemblies 30 are connected to the circuit board 52, and the second conductive ends 30b of each electrode assembly 30 may be electrically connected to the circuit board 52. The circuit board 52 is further electrically connected to the power supply component 40. The mechanical switch 53 is connected to circuit board 52. The mechanical switch 53 may be a toggle switch, which can be switched between at least two positions under an external force, and each position corresponds to one liquid storage component 200. When the mechanical switch 53 is switched to one position, electronic circuits inside the circuit board 52 are connected to one electrode assembly 30 and the power supply component 40, respectively (i.e., one liquid storage component 200 is selected to operate by turning the mechanical switch 53, and the liquid storage component 200 communicates with the inhalation port P0). Also, when the airflow switch 51 detects a change of airflow or pressure, the electrical signal generated by the airflow switch 51 causes the electronic circuits in the circuit board 52 to conduct, thereby electrically connecting the power supply component 40 to the electrode assembly 30. That is, the circuit board 52 can electrically connect the power supply component 40 to one electrode assembly 30 based on the detection result of the airflow switch 51 and the position of the mechanical switch 53, such that the power supply component 40 is electrically connected to one liquid storage component 200 through the electrode assembly 30, while the storage component 200 communicating with the inhalation port P0. For example, taking the main shell 10 having two receiving chambers 11 for receiving two liquid storage components 200 as an example, the mechanical switch 53 can switch between two positions. When the mechanical switch 53 is turned to the left position shown in FIG. 10 and the airflow switch 51 detects a change of airflow or pressure, the power supply component 40 is electrically connected to the liquid storage component 200 on the left side shown in FIG. 10; when the mechanical switch 53 is turned to the right position shown in FIG. 10 and the airflow switch 51 detects a change of airflow or pressure, the power supply component 40 is electrically connected to the liquid storage component 200 on the right side shown in FIG. 10.

As shown in FIG. 10, the position of the circuit switching component 50 may also be changed. For example, the circuit board 52 may be located in the first receiving area 151, and the mechanical switch 53 may be exposed from the bottom shell 60, thereby facilitating the turning of the mechanical switch 53 by the user.

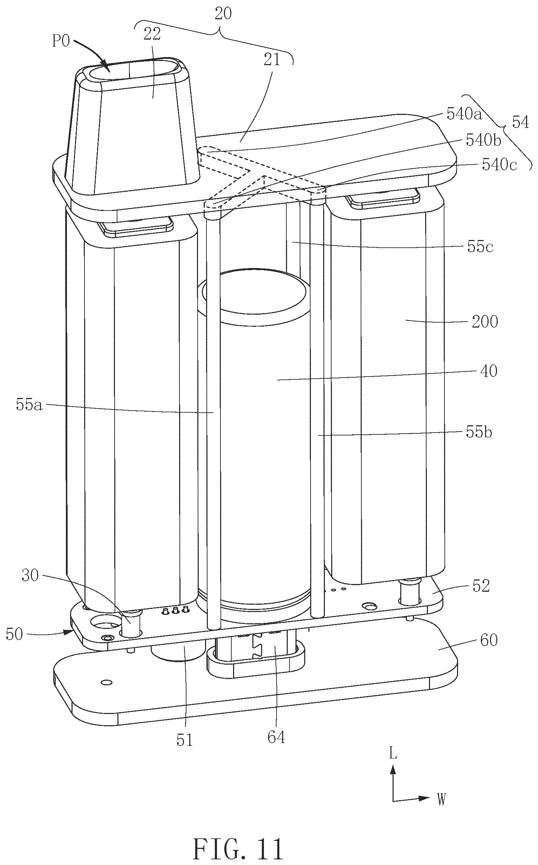

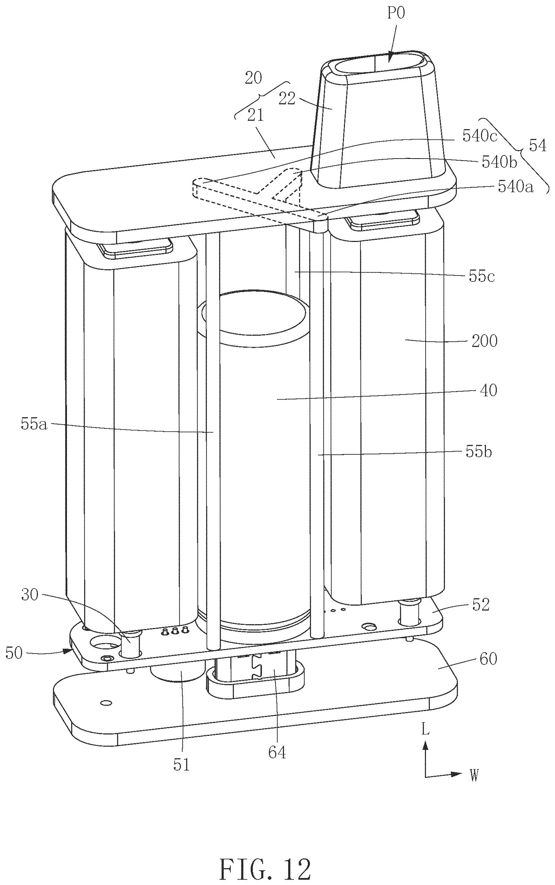

The structure of the circuit switching component 50 may also be changed. As shown in FIGS. 11 and 12, in other embodiments, the circuit switching component 50 may include an airflow switch 51, an electrical connection component 54, a circuit board 52, and at least two third electrodes. There is only one airflow switch, which simultaneously communicates with the first air passage P1 of each liquid storage component 200. The electrical connection component 54 is provided on the mouthpiece 20. Since the mouthpiece 20 is movably connected to the first end 10a, when the mouthpiece 20 moves relative to the main shell 10, the electrical connection component 54 can move together with the mouthpiece 20 relative to the main shell 10. The electrode assemblies 30 are electrically connected to the circuit board 52. The electrode assembly 30 may be installed on the circuit board 52, and the second conductive ends 30b of the electrode assembly 30 is electrically connected to the circuit board 52. The circuit board 52 is further electrically connected to the power supply component 40. The third electrodes are mounted on circuit board 52 and extend towards electrical connection component 54. In some embodiments, when there are two receiving chambers 11 for receiving two liquid storage components 200, the number of third electrodes may be three. The electrical connection component 54 can selectively conduct two third electrodes when the mouthpiece 20 moves relative to the main shell 10. The two third electrodes that are conducted can select one liquid storage component 200 to operate, while the liquid storage component 200 communicating with the inhalation port P0. When the airflow switch 51 detects a change of airflow or pressure, the electrical signal generated by the airflow switch 51 electrically connects the power supply component 40 to one electrode assembly 30, thereby enabling the power supply component 40 to be electrically connected to one liquid storage component 200 through the electrode assembly 30, while the liquid storage component 200 communicating with the inhalation port P0. For example, taking the main shell 10 having two receiving chambers 11 for receiving two liquid storage components 200 as an example, there are three third electrodes, including an electrode 55a, an electrode 55b, and an electrode 55c. Accordingly, the electrical connection component 54 includes three electrical connection terminals, including an electrical connection terminal 540a, an electrical connection terminal 540b, and an electrical connection terminal 540c. As shown in FIG. 11, when the inhalation port P0 of the mouthpiece 20 corresponds to the liquid storage component 200 on the left side shown in FIG. 11, the electrical connection terminals 540b, 540c of the electrical connection component 54 are connected to the electrodes 55a and 55b, respectively. When the airflow switch 51 detects a change of airflow or pressure at this time, the power supply component 40 is electrically connected to the liquid storage component 200 on the left side shown in FIG. 11. As shown in FIG. 12, when the inhalation port P0 of the mouthpiece 20 corresponds to the liquid storage component 200 on the right side shown in FIG. 12, the electrical connection terminals 540a, 540c of the electrical connection component 54 are connected to the electrodes 55b and 55c, respectively. When the airflow switch 51 detects a change of airflow or pressure at this time, the power supply component 40 is electrically connected to the liquid storage component 200 on the right side shown in FIG. 12.

In other embodiments, when there are two receiving chambers 11 for receiving two liquid storage components 200, the number of third electrodes may also be two. The two third electrodes correspond to the two liquid storage components 200, respectively. In this case, the electrical connection component 54 can selectively conduct or isolate the two third electrodes from each other when the mouthpiece 20 moves relative to the main shell 10, thereby selecting one liquid storage component 200 to operate (i.e., one liquid storage component 200 communicating with the inhalation port P0). For example, one liquid storage component 200 is selected to operate based on the electrical signal generated when the two third electrodes are conducted, and another liquid storage component 200 is selected to operate when the two third electrodes are isolated. When the airflow switch 51 detects a change of airflow or pressure at this time, the electrical signal generated by the airflow switch 51 electrically connects the power supply component 40 to one electrode assembly 30, thereby enabling the power supply component 40 to be electrically connected to one liquid storage component 200 through the electrode assembly 30, while the liquid storage component 200 communicating with the inhalation port P0.

In other embodiments, when there are two receiving chambers 11 and two third electrodes, a wire (not shown) may also be connected between the circuit board 52 and the electrical connection component 54. In this case, the electrical connection component 54 can selectively conduct one third electrode when the mouthpiece 20 moves relative to the main shell 10, thereby selecting one liquid storage component 200 to operate (i.e., one liquid storage component 200 communicating with the inhalation port P0 and corresponding to the conducted third electrode). When the airflow switch 51 detects a change of airflow or pressure at this time, the electrical signal generated by the airflow switch 51 electrically connects the power supply component 40 to one electrode assembly 30, thereby enabling the power supply component 40 to be electrically connected to one liquid storage component 200 through the electrode assembly 30, while the liquid storage component 200 communicating with the inhalation port P0.

As shown in FIGS. 11 and 12, the position of the circuit switching component 50 may also be changed. For example, the circuit board 52 may be located in the first receiving area 151, and the third electrode may be mounted on the circuit board 52 and extend into the second receiving area 152. The circuit switching component 50 may be a built-in switch at this time, which is not exposed from the main shell 10 or the bottom shell 60.

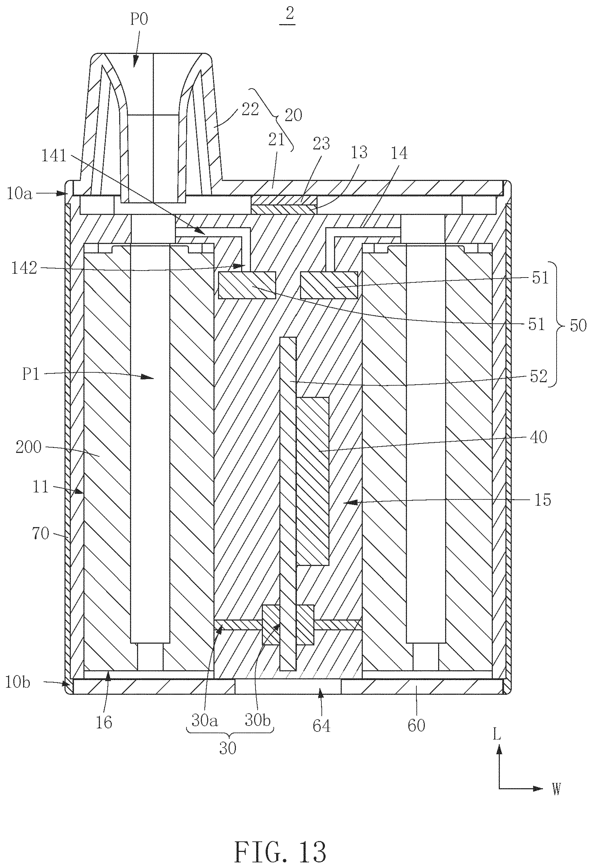

Referring to FIG. 13, an atomization device 2 is further provided according to another embodiment of the present application. The circuit switching component 50 includes at least two airflow switches 51 and a circuit board 52, but the positions of the electrode assemblies 30 and the circuit switching component 50 are different from those of the atomization device 1 shown in FIG. 5.

As shown in FIG. 13, in some embodiments, the electrode assembly 30, the power supply component 40, and the circuit switching component 50 are disposed within the main shell 10 and located between the receiving chambers 11. For example, the main shell 10 is further provided with a receiving space 15 between the receiving chambers 11. The electrode assemblies 30, the power supply component 40, and the circuit switching component 50 are located in the receiving space 15. The first conductive ends 30a of each electrode assembly 30 extends from the receiving space 15 into the corresponding receiving chamber 11, and are electrically connected to the corresponding liquid storage component 200. As such, the internal structure of the atomization device 2 is compact, thereby improving the space utilization. The main shell 10 is further provided with at least two first air branch passages 14, which correspond to the at least two airflow switches 51, respectively. The first air branch passage 14 includes a first end portion 141 and a second end portion 142. The first end portion 141 of each first air branch passage 14 is connected to the first air passage P1 of one liquid storage component 200, and the second end portion 142 of each first air branch passage 14 is connected to one airflow switch 51. Therefore, when the user inhales at the inhalation port P0 of the mouthpiece 20, a change of airflow or pressure in one first air branch passage 14, which connects to the liquid storage component 200 communicating with the inhalation port P0, will be detected by the airflow switch 51 connected to the first air branch passage 14. Therefore, the electronic circuits in the circuit board 52 conducts, which electrically connects the power supply component 40 to one electrode assembly 30, such that the power supply component 40 is electrically connected to one liquid storage component 200 through the electrode assembly 30, while the liquid storage component 200 communicating with the inhalation port P0.

As shown in FIG. 13, the main shell 10 may further include at least two second openings 16 located at the second end 10b, which correspond to the at least two receiving chambers 11, respectively. The bottom shell 60 is detachably connected to the second end 10b. When the bottom shell 60 is removed from the second end 10b, the liquid storage components 200 are exposed from the second openings 16, thereby facilitating the replacement of the liquid storage components 200.

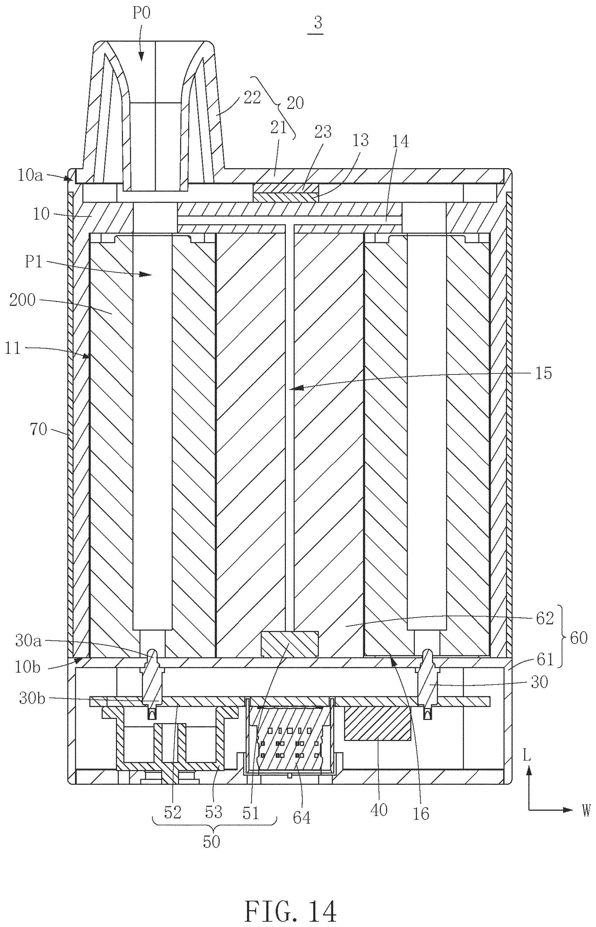

Referring to FIG. 14, an atomization device 3 is further provided according to yet another embodiment of the present application. The circuit switching component 50 includes an airflow switch 51, a circuit board 52, and a mechanical switch 53, but the positions of the electrode assemblies 30, the power supply component 40, and the circuit switching component 50 are different from those of the atomization device 1 shown in FIG. 10.

As shown in FIG. 14, in some embodiments, the power supply component 40, the electrode assemblies 30, and the circuit switching component 50 are located in the bottom shell 60. The first conductive ends 30a of each electrode assembly 30 extend from the bottom shell 60 into the corresponding receiving chamber 11, and are electrically connected to the corresponding liquid storage component 200. The main shell 10 is further provided with a first air branch passage 14, which simultaneously communicates with the first air passage P1 of each liquid storage component 200. The bottom shell 60 is detachably connected to the second end 10b. The bottom shell 60 includes a bottom shell body 61 connected to the second end 10b and a bottom shell seat 62 fixed to the bottom shell body 61. The power supply component 40, the electrode assemblies 30, and the circuit switching component 50 are located in the bottom shell body 61. The bottom shell seat 62 is provided with a second air branch passage 63 communicating with the first air branch passage 14. The second air branch passage 63 is also connected to the airflow switch 51. Therefore, when the user inhales at the inhalation port P0 of the mouthpiece 20, the airflow switch 51 can detect a change of airflow or pressure in the airflow channel.

As shown in FIG. 14, the main shell 10 may further include at least two second openings 16 located at the second end 10b, which correspond to the at least two receiving chambers 11, respectively. When the bottom shell 60 is removed from the second end 10b, the liquid storage components 200 are exposed from the second openings 16, thereby facilitating the replacement of the liquid storage components 200.

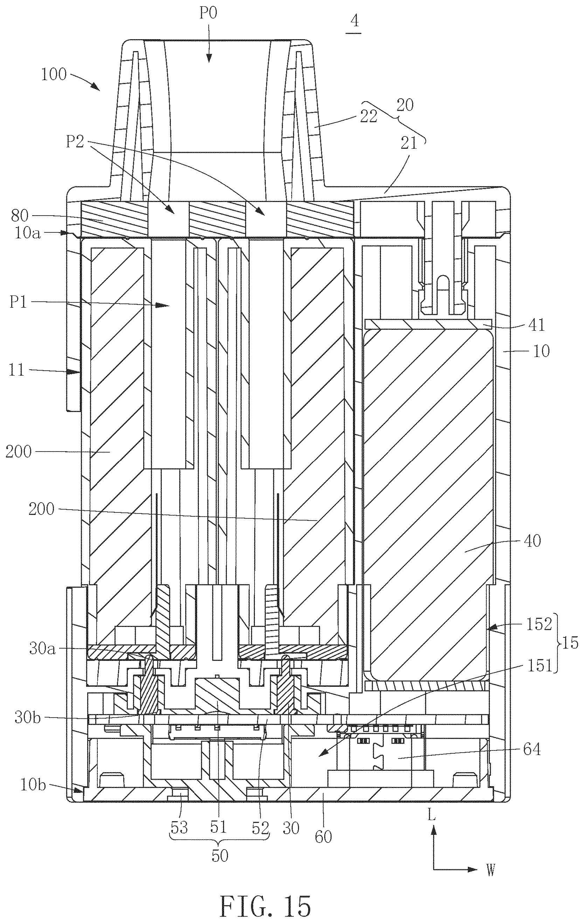

Referring to FIG. 15, an atomization device 4 is further provided according to yet another embodiment of the present application. The difference between the atomization device 4 at the atomization device 1 mentioned above is that the mouthpiece 20 is fixed to the first end 10a. When viewed from the length direction L, the inhalation port P0 covers the first air passage P1 of each liquid storage component 200. Therefore, each liquid storage component 200 can shares the single mouthpiece 20 to form an air channel, and the aerosol from the first air passage P1 of each liquid storage component 200 can be delivered toward the mouthpiece 20, thereby reducing the bends in the air channel and reducing the requirements for flavor adjustment.

As shown in FIG. 15, in some embodiments, the power supply component 40 is located in the main shell 10. Along the width direction W, the power supply component 40 is located at a same side of the receiving chambers 11, thereby enabling the inhalation port P0 to cover the first air passages P1 of each liquid storage component 200. The electrode assembly 30 and the circuit switching component 50 are located on a side of the bottom shell 60 facing the mouthpiece 20. For example, the main shell 10 is further provided with a receiving space 15. The receiving space 15 is divided into a first receiving area 151 and a second receiving area 152 communicating with the first receiving area 151. The first receiving area 151 is located at a same side of the receiving chambers 11 along the length direction L, and is closer to the second end 10b than the receiving chambers 11. The second receiving area 152 is located at a same side of the receiving chambers 11 along the width direction W. The power supply component 40 is located in the second receiving area 152. The electrode assemblies 30 and the circuit switching component 50 are located in the first receiving area 151. FIG. 15 shows that the circuit switching component 50 of the atomization device 4 includes an airflow switch 51, a circuit board 52, and a mechanical switch 53. It can be understood that the circuit switching component 50 shown in FIG. 5 is also applicable to the atomization device 4.

As shown in FIG. 15, in some embodiments, the atomization device 4 may further include a sealing component 80 provided in the mouthpiece 20. The sealing component 80 is provided with at least two second air passages P2, and second air passage P2 communicates with the first air passage P1 of one liquid storage component 200. The inhalation port P0, the second air passage P2, and the first air passage P1 cooperatively form the airflow channel. The sealing component 80 can seal the gap between the main shell 10 and the mouthpiece 20, thereby reducing the leakage of the aerosolizable substance of the liquid storage component 200 from the above gap and the leakage of the aerosol from the above gap. The sealing component 80 may be made of silicone or rubber.

Even though information of the present embodiments has been set forth in the foregoing description, one having ordinary skill in the art would realize that various changes, modifications, or substitutions may be made to the embodiments without departing from the principle and spirit of the present application. The scope of the present application is limited by the appended claims and their equivalents.

## Claims

1. An atomization assembly (100), **characterized by** comprising:
a main shell (10) comprising a first end (10a) and a second end (10b) arranged opposite to the first end (10a) along a length direction (L) of the atomization assembly (100), wherein the main shell (10) is provided with at least two receiving chambers (11), each of the receiving chambers (11) is configured to receive a liquid storage component (200) therein;
a mouthpiece (20) connected to the first end (10a) and provided with an inhalation port (P0), the inhalation port (P0) configured to correspond to the liquid storage component (200) in at least one of the receiving chambers (11);
at least two electrode assemblies (30) configured to correspond one-to-one to and electrically connected to the liquid storage components (200) in the at least two receiving chambers (11);
a power supply component (40); and,
a circuit switching component (50) electrically connected to the power supply component (40), wherein the circuit switching component (50) selectively connecting the power supply component (40) to one of the at least two electrode assemblies (30), thereby establishing an electrical connection between the power supply component (40) and the liquid storage component (200) corresponding to the inhalation port (P0).

2. The atomization assembly (100) according to claim 1, **characterized in that**, the mouthpiece (20) is movably connected to the first end (10a), the liquid storage component (200) is provided with a first air passage (P1), and the inhalation port (P0) selectively communicates with the first air passage (P1) of the liquid storage component (200) in one of the at least two receiving chambers (11) when the mouthpiece (20) moves relative to the main shell (10).

3. The atomization assembly (100) according to claim 1, **characterized in that**, the mouthpiece (20) is fixed to the first end (10a), the liquid storage component (200) is provided with a first air passage (P1), and when viewed along the length direction (L), the inhalation port (P0) is configured to cover the first air passages (P1) of the liquid storage components (200) (200) in the at least two receiving chambers (11).

4. The atomization assembly (100) according to claim 2 or 3, **characterized in that**, the circuit switching component (50) comprises:
at least two airflow switches (51) being arranged to correspond one-to-one to the at least two receiving chambers (11), and the at least two airflow switches (51) configured to detect a change of airflow or pressure in the first air passages (P1) of the liquid storage components (200) in the at least two receiving chambers (11), respectively; and
a circuit board (52), the electrode assemblies (30) electrically connected to the circuit board (52), the circuit board (52) further electrically connected to the power supply component (40) and the at least two airflow switches (51), wherein the circuit board (52) being configured to electrically connect the power supply component (40) to one of the at least two electrode assemblies (30) based on a detection result of the airflow switches (51).

5. The atomization assembly (100) according to claim 2 or 3, **characterized in that**, the circuit switching component (50) comprises:
an airflow switch (51) configured to detect a change of airflow or pressure in the first air passages (P1) of the liquid storage components (200) in the at least two receiving chambers (11);
a circuit board (52), the electrode assemblies (30) electrically connected to the circuit board (52), and the circuit board (52) further electrically connected to the power supply component (40); and,
a mechanical switch (53) connected to the circuit board (52) and configured to switch between at least two positions, the circuit board (52) configured to electrically connect the power supply component (40) to one of the at least two electrode assemblies (30) based on a detection result of the airflow switch (51) and the position of the mechanical switch (53).

6. The atomization assembly (100) according to claim 2, **characterized in that**, the circuit switching component (50) comprises:
an airflow switch (51) configured to detect a change of airflow or pressure in the first air passages (P1) of the liquid storage components (200) in the at least two receiving chambers (11);
an electrical connection component (54) located in the mouthpiece (20);
a circuit board (52), the electrode assemblies (30) electrically connected to the circuit board (52), and the circuit board (52) further electrically connected to the power supply component (40); and,
at least two electrodes installed on the circuit board (52) and extending towards the electrical connection component (54), wherein the electrical connection component (54) selectively conducts at least one of the electrodes when the mouthpiece (20) moves relative to the main shell (10), the circuit board (52) is configured to electrically connect the power supply component (40) to one of the at least two electrode assemblies (30) based on a detection result of the airflow switch (51) and the conducted electrode.

7. The atomization assembly (100) according to claim 4, **characterized in that**, the power supply component (40) is disposed within the main shell (10) and located between the at least two receiving chambers (11), wherein the atomization assembly (100) further comprises a bottom shell (60) detachably connected to the second end (10b), and the electrode assemblies (30) and the circuit switching component (50) are located at a side of the bottom shell (60) facing the mouthpiece (20).

8. The atomization assembly (100) according to claim 4, **characterized in that**, the power supply component (40), the electrode assemblies (30), and the circuit switching component (50) are disposed within the main shell (10) and located between the at least two receiving chambers (11), the main shell (10) is further provided with at least two first air branch passages (14), each of the at least two first air branch passages (14) comprises a first end portion (141) and a second end portion (142), the first end portion (141) is configured to communicate with the first air passage (P1) of the liquid storage component (200) in one of the at least two receiving chambers (11), and the second end portion (142) is connected to one of the at least two airflow switches (51).

9. The atomization assembly (100) according to claim 5, **characterized in that**, the power supply component (40) is disposed within the main shell (10) and located between the at least two receiving chambers (11), the atomization assembly (100) further comprises a bottom shell (60) detachably connected to the second end (10b), and the electrode assemblies (30) and the circuit switching component (50) are located at a side of the bottom shell (60) facing the mouthpiece (20).

10. The atomization assembly (100) according to claim 5, **characterized in that**, the atomization assembly (100) further comprises a bottom shell (60) detachably connected to the second end (10b), the power supply component (40), the electrode assemblies (30), and the circuit switching component (50) are located in the bottom shell (60), the main shell (10) is further provided with a first air branch passage (14), the first air branch passage (14) is configured to communicate with the first air passages (P1) of the liquid storage components (200) in the at least two receiving chambers (11), the bottom shell (60) is provided with a second air branch passage (63) communicating with the first air branch passage (14), and the second air branch passage (63) is further connected to the airflow switch (51).

11. The atomization assembly (100) according to any one of claims 2 and 4-10, **characterized in that**, the mouthpiece (20) is provided with a first magnetic component (23), the main shell (10) is provided with a second magnetic component (13), and the first magnetic component (23) is detachably connected to the second magnetic component (13).

12. The atomization assembly (100) according to any one of claims 2 and 4-10, **characterized in that**, the mouthpiece (20) is provided with one of a buckle and a slot, the main shell (10) is provided with the other of the buckle and the slot, and the buckle is detachably connected to the slot.

13. The atomization assembly (100) according to any one of claims 3-5 and 7-10, **characterized in that**, the power supply component (40) is located in the main shell (10), the power supply component (40) is located at a side of the at least two receiving chambers (11) along a width direction (W) perpendicular to the length direction (L), the atomization assembly (100) further comprises a bottom shell (60) detachably connected to the second end (10b), and the electrode assemblies (30) and the circuit switching component (50) are located at a side of the bottom shell (60) facing the mouthpiece (20).

14. An atomization device (1, 2, 3, 4), **characterized by** comprising:
the atomization assembly (100) according to any one of claims 1 to 13; and
at least two liquid storage components (200) received in the at least two receiving chambers (11) of the atomization assembly (100), respectively.
